# EUROPEAN PATENT SPECIFICATION

(11) **EP 1 353 179 B1**
(45) Date of publication and mention of the grant of the patent: **12.09.2007**
(21) Application number: 02715712.2
(22) Date of filing: 09.01.2002
(51) Int. Cl.: G01N 33/53, G01N 33/566, G01N 33/483, G01N 33/543, G01N 33/553, G01N 37/00, G01N 21/64

(54) **FLUORESCENT ANALYSIS ELEMENT WITH THE USE OF METALLIC NANOWELL AND PROCESS FOR PRODUCING THE SAME**
FLUORESZENSANALYSEELEMENT MIT DER ANWENDUNG EINES METALLISCHEN NANOLOCHS UND VERFAHREN ZUR HERSTELLUNG DESSELBEN
ELEMENT D'ANALYSE PAR FLUORESCENCE AU MOYEN D'UN NANOPUITS METALLIQUE ET PROCEDE DE PRODUCTION DE CET ELEMENT

(30) Priority: 12.01.2001 JP 2001005041
(43) Date of publication of application: 15.10.2003
(73) Proprietor: Japan Science and Technology Corporation, Kawaguchi-shi, Saitama 332-0012 (JP)
(72) Inventor: ISHIDA, Akito, Minou-shi, Osaka 562-0031 (JP)
(74) Representative: Müller-Boré & Partner Patentanwälte
(86) International application number: PCT/JP2002/000026
(87) International publication number: WO 2002/056012

(56) References cited:
- EP-A- 0 872 733
- EP-A- 0 950 718
- JP-A- 10 307 141
- JP-A- 10 311 831
- JP-A- 11 332 595
- JP-A- 11 344 437
- JP-A- 2000 249 706
- JP-A- 2001 021 565
- BARNES ET AL: "ENHANCED FLUORESCENCE YIELDS THROUGH CAVITY QUANTUM-ELECTRODYNAMIC EFFECTS IN MICRODROPLETS" JOURNAL OF THE OPTICAL SOCIETY OF AMERICA, vol. 11, July 1994 (1994-07), pages 1297-1304, XP002274349
- LIU Y ET AL: "FLUORESCENCE ENHANCEMENT FROM AN ARRAY OF SUBWAVELENGTH METAL APERTURES" OPTICS LETTERS, OPTICAL SOCIETY OF AMERICA, WASHINGTON, US, vol. 28, no. 7, 1 April 2003 (2003-04-01), pages 507-509, XP001160143 ISSN: 0146-9592

## Description

### TECHNICAL FIELD

The present invention relates to a new element for fluorescence analysis useful in various fields such as biochemistry and clinical or environmental analysis, and also relates to a method of manufacturing the element.

### BACKGROUND

In conventional immunity or genetic analysis, objective antigen or DNA has been detected or measured by coupling interaction between antigen and antibody or between complementary DNAs. Especially, fluorescence analysis, whereby the coupling interaction is detected as fluorescence, is applied to broad fields involving biochemical researches and clinical analysis due to high sensitivity.

Various processes have been proposed so far as fluorescence analysis, but basically executed by steps as follows:
(1) Prepare a plate chemically modified with an antibody or DNA, which is strongly coupled with an objective antigen or DNA, on its surface.
(2) Modify the objective antigen or DNA by adding a fluorescence-labeling reagent to a sampled specimen containing the objective antigen or DNA.
(3) Couple the objective antigen or DNA with the antibody or complementary DNA by immersion of the plate in the sampled specimen, and sufficiently wash a surface of the plate so as to remove uncoupled components from the surface.
(4) Detect a fluorescent signal from the objective antigen or DNA bonded to the plate by photo-excitation, and calculate concentration of the objective antigen or DNA from intensity of the fluorescence.

The process involving the steps (1) and (2) is too simple, so sensitivity is commonly improved by combination of various procedures. Some influential procedures have been established so far for improvement of sensitivity. A multi-welled plate having 96-1536 wells is also used as a substrate for analysis, wherein a series of various processes and measurement in one well are completely automated so as to perform analysis operation with high throughput. A DNA tip, which has DNAs accumulatively fixed as spots of 200 µm or so on its surface, is sometimes used especially for genetic analysis, in order to achieve extremely high throughput.

By the way, a number of specimens become more and more in correspondence with rapid development and enlargement of researches on fundamental science and extreme increase of various virus and allergic diseases on clinical analysis. Although integration of a multi-welled plate is developed as noted by practical use of 1536 wells, a situation difficult to cope with demands is clearly forecast in near future, as far as wells of visible size are used for analysis.

Even an advanced DNA tip with high sensitivity has a spot size of 150 µm or so at smallest, so its whole size is several centimeter square. Accounting exponential increase in number of genetic specimens for analysis, a conventional integration is extremely insufficient. In this sense, there is a strong demand for provision of micro-tips, wherein nano-wells of a several µm or smaller in size are integrated.

However, decrease of sample volume leads to decrease in number of excited molecules and also exponential increase of errors caused by turbulent factors, resulting in significant decrease of fluorescent intensity. Decrease of sample volume also causes discoloration of a fluorescent reagent, i.e. biggest trouble in results of fluorescence analysis. In this regard, the following requirements (a) to (c) shall be fulfilled in order to make fluorescence analysis with nano-wells available for practical use. But, it is very difficult to individually solve the requirements, since each requirement correlates mutually and strongly with the other.
(a) To suppress interaction between a fluorescent reagent and such discoloration-promoting stuffs such as oxygen, by fixing and enclosing a reagent or sample for detection in a minute space closed as much as possible.
(b) To transmit an energy of exciting light to a fluorescent molecule with high efficiency.
(c) To introduce generated fluorescence to a detector with high efficiency.

### SUMMARY OF THE INVENTION

The present invention aims at provision of a new element which fulfills all the requirements (a) to (c), by using holes of several hundreds nanometer in diameter formed in a metal film of from several hundreds nanometer to several microns in thickness as nano-wells for fluorescence analysis.

The element for fluorescence analysis proposed by the present invention comprises a metal film deposited on a surface of a substrate, nano-wells formed in the metal film and an active group or DNA fixed to a bottom of each nano-well. An antigen coupled to the active group or an DNA is hybridized with the nano-well fixed DNA, and fluorescent reagent bonded to the antigen or the DNA is excited by a luminescent energy so as to emit fluorescence.

The element is manufactured by processing a substrate with a silane-coupling reagent, depositing a metal film on the substrate, and fixing an active group or DNA to a bottom of each nano-well. The nano-wells are formed by a projection method of depositing Au, Ag or the like from a vapor phase, or by such a direct writing method as electron beam lithography, photolithography or laser beam irradiation.

### BRIEF DESCRIPTION OF THE DRAWINGS

Fig. 1 is a flow chart for explaining formation of nano-wells in a metal film deposited on a substrate.
Fig. 2 is a flow chart for explaining modification of a bottom of a nano-well with an antibody.
Fig. 3A is a graph illustrating fluorescent spectrum of Rhodamine 6G coupled to an antibody.
Fig. 3B is a graph for explaining an effect of Rhodamine 6G on intensity of fluorescence.
Fig. 4 is a flow chart for explaining modification of a nano-well with avidin.
Fig. 5A is a model for explaining biotinylation of DNA for detection.
Fig. 5B is a model for explaining interaction between a modified nano-well and avidin.
Fig. 6 is a flow chart for explaining genetic analysis by hybridization of DNA.

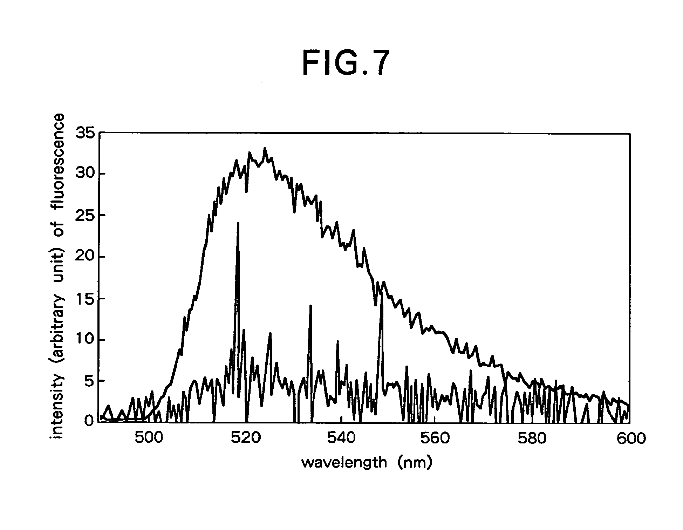

Fig. 7 is a graph illustrating spectrum of fluorescence emitted from a fluorescent reagent, which modifies hybridized DNA, by excitation with a light energy.

### BEST MODES OF THE INVENTION

An element for fluorescence analysis newly proposed by the present invention has such a substrate as quartz, glass, sapphire, silicon having an oxidized surface, a diamond film or a metal sheet. A metal film is deposited with thickness of from several hundreds nanometer to several micrometer on a surface of the substrate. Holes of several hundreds nanometers in diameter present in the thin metal film are used as nano-wells. The metal film is preferably Au or Ag, since the Au or Ag film can be easily modified with various proteins and nucleic acids. The Au or Ag film is also appropriate for provision of a field for immunity or genetic analysis, and facilitates fixation of a detecting reagent or sample in nano-wells (hereinafter referred to as "metallic nano-well") present therein.

The metallic nano-well has a capillary force to suck a droplet, which comes in contact with a surface of the metal film, in its inner space. Due to the capillary force, a detecting reagent or sample is fixed to the metallic nano-well with ease, and enclosed in the minute space. Consequently, contact area of the reagent or sample with air is remarkably reduced, and discoloration of fluorescence is inhibited.

Furthermore, the metallic nano-well on the metal film advantageously promotes emission of fluorescence from a fluorescent reagent. According to a conventional fluorescence analysis, wherein transparency of a substrate and intensity of a light source are adjusted at possible highest levels to improve excitation efficiency, decrease of sample volume means troubles on applicability to practical use. On the other hand, the inventive metallic nano-well improves excitation efficiency according to a principle basically different from the conventional fluorescence analysis, due to the Au or Ag film with low light transmissivity formed on the substrate. The inventors suppose an effect of the metal film on improvement of excitation efficiency as follows:

When a smooth surface of an Au or Ag film is irradiated with light, most of the irradiating light is consumed by reflection or transmission. On the other hand, an energy of light, which is dispatched to a metal film having metallic nano-wells of diameter near wavelength of exciting light, is converted and amplified to a surface plasmon electromagnetic field with hundreds of times electromagnetic field strength, and the strong surface plasmon electromagnetic field is localized inside the metallic nano-well. The surface plasmon electromagnetic field has characteristics similar to light and excites a fluorescent sample. In this sense, the metallic nano-well is regarded as an antenna for condensation of an energy of exciting light therein.

The sample enclosed in the metallic nano-well is exposed to the intensified surface plasmon electromagnetic field and excited by an energy from the surface plasmon electromagnetic field. Consequently, the fluorescent sample is excited with extremely high efficiency, compared with a conventional exciting process wherein a sample is directly irradiated with light.

The metallic nano-well is also advantageous for improvement of detection efficiency. Since any of detecting reagents and samples for is a liquid with refractive index similar to water, the metallic nano-well serve as a waveguide as increase of a ratio (an aspect ratio) of diameter of the metallic nano-well to thickness of the metal film. Due to the function as a waveguide, fluorescence emitted from the sample inside the metallic nano-well is effectively condensed and guided from an aperture of the metallic nano-well to a detector.

Analysis can be performed with surprisingly high density due to the above-mentioned features of the metallic nano-well, and efficient excitation is well balanced with efficient detection. As a result, intensity of exciting light for production of a proper fluorescent signal can be reduced to a remarkably lower level compared with a conventional fluorescence analysis, so as to enable analysis with further elevated accuracy and sensitivity without discoloration of fluorescence.

### Example 1

The present invention will be apparent from an example applied to immunoassay.

### Preparation of metallic nano-well

This example adopts a projection process for forming holes useful as metallic nano-wells in a metal film, but other processes such as electron beam lithography, photolithography or laser pattern generation are also applicable for formation of metallic nano-wells. According to the projection process, an object is located between an evaporation source and a substrate, and a shape of the object is projected to a metal film deposited on the substrate.

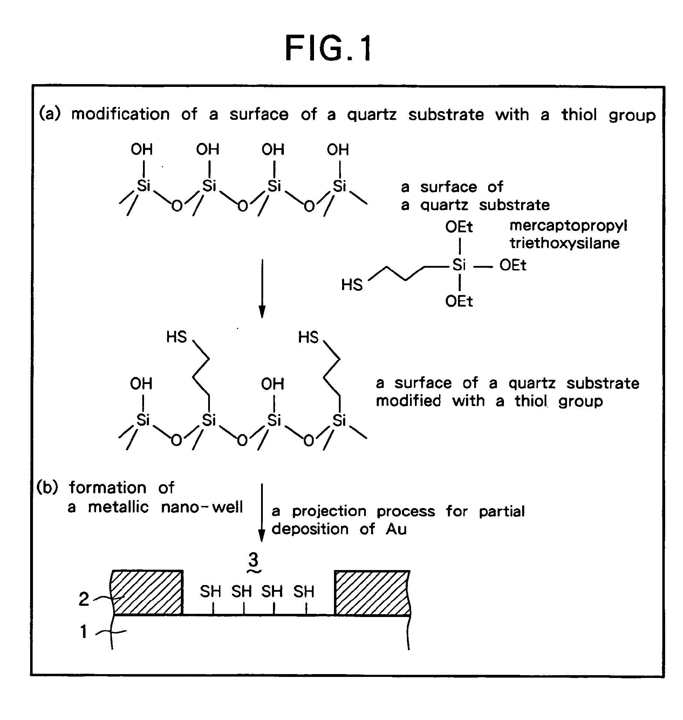

A surface of a quartz substrate 1 was processed with a silane-coupling reagent having a thiol group at its terminal, to form a monolayer film (indicated by (a) in Fig. 1) having the thiol group on a surface of the quartz substrate 1 by substitutional reaction between the thiol group and a OH group on the surface of the quartz substrate 1. Mercaptopropyl triethoxysilane was used as the silane-coupling reagent, but there are no restriction on kinds of the silane-coupling reagent as far as it has a thiol group at its terminal. The monomolecular film having the thiol group assures rigid fixation of a metal film onto the quartz substrate 1 by vapor deposition in the succeeding step and also serves as an active part during modifying a bottom of a metallic nano-well with a molecule.

A dilute liquid, with dispersed polystyrene latex spheres of 480 nm in diameter therein, was applied to the processed surface of the quartz substrate 1. After a solvent was gradually vaporized from the liquid, the latex spheres remained in scattered state on the surface of the quartz substrate 1. Thereafter, a metal film 2 of 70 nm in thickness was formed on the quartz substrate 1 by vapor deposition of Au. Other metals such as Ag, Cu and Al are also useful as film material instead of Au, as far as surface plasmon resonance occurs from the metal film.

The quartz substrate 1 coated with the metal film 2 was immersed in an organic solvent for dissolution removal of the latex spheres from the surface of the quartz substrate 1. Traces of the latex spheres remained as metallic nano-wells 3 in the metal film 2, and the monomolecular film with the thiol group fixed to the surface of the quartz substrate 1 was exposed at a bottom of each metallic nano-well 3 (indicated by (b) in Fig. 1).

Polystyrene latex spheres are selected from those of optimum diameter in response to wavelength of exciting light, properties of a sample, arrangement of a detector system and so on, since the metallic nano-wells formed in this way have inner diameter corresponding to diameter of the latex spheres. The metal film is also controlled to optimum thickness in response to wavelength of exciting light, properties of a sample, arrangement of a detector system and so on.

### Modification of a bottom of a metallic nano-well

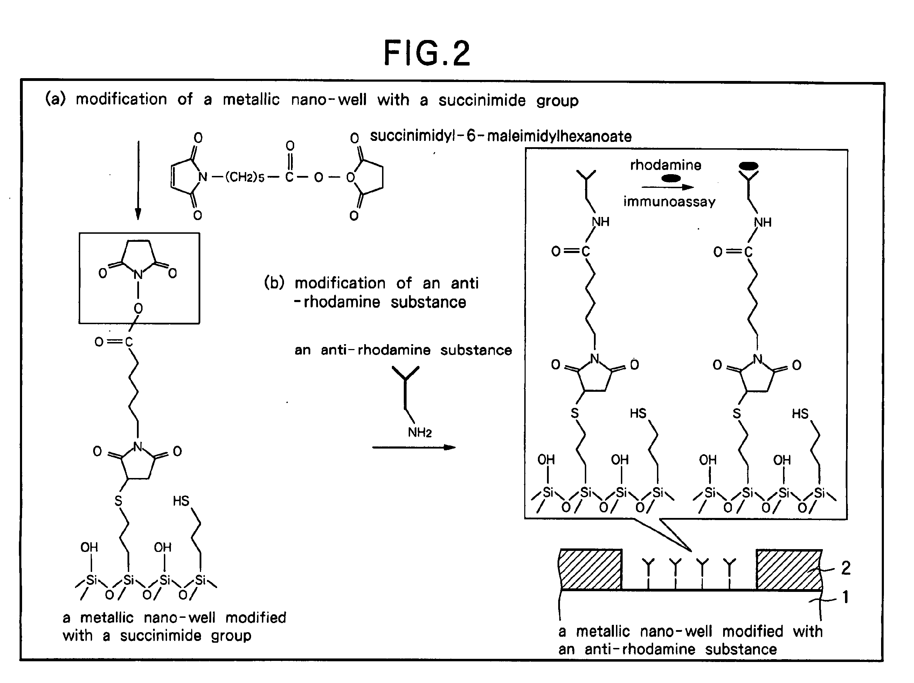

In order to prove capability of immunoassay inside the metallic nano-well 3, a bottom of the metallic nano-well 3 was modified with a molecule as follows: At first, the quartz substrate 1 was completely immersed in a solution of succinimidyl-6-maleimidylhexanoate and then sufficiently washed, to modify a bottom of the metallic nano-well 3 with a succinimidyl group (indicated by (a) in Fig. 2) by interaction between succinimidyl-6-maleimidylhexanoate and the thiol group at the bottom of the metallic nano-well 3. The modified quartz substrate 1 was immersed in a solution of an anti-rhodamine antibody, so as to fix the anti-rhodamine antibody to the bottom of the metallic nano-well 3 by reaction between the succinimidyl group at the bottom of the metallic nano-well 3 and an amino group on the anti-rhodamine antibody.

Various antibodies and reagents can be used for fixation, instead of succinimidyl-6-maleimidylhexanoate and anti-rhodamine antibody. For instance, a double antibody may be used for the purpose. The above-mentioned process of immersing the quartz substrate 1 in the chemical solution is useful for modification of all the metallic nano-wells 3 with the same antibody, but other processes may be adopted instead of the immersion process. For instance, droplets are handled with a micropipette, or droplets are patterned by an ink-jet printer, in order to individually modify the metallic nano-wells 3 with various kinds of antibodies.

### Reaction between antigen and antibody

A 1µM solution of Rhodamine 6G was dropped on the metal film 2 having the metallic nano-wells 3 modified with the anti-rhodamine antibody and held as such for 15 minutes. Thereafter, the metal film 2 was washed. Rhodamine 6G was introduced into the anti-rhodamine antibody.

### Generation of a surface plasmon electromagnetic field

### and observation of fluorescence

The metal film 2 processed as above-mentioned was fixed on a stage of a fluorescence microscope with coaxial illuminator and irradiated with an Ar-laser beam of 488 nm. A surface plasmon electromagnetic field was generated by laser irradiation, and fluorescence was emitted from Rhodamine 6G inside the metallic nano-well 3. The fluorescence was observed by the fluorescence microscope. During observation, a view is squeezed with a pinhole, and fluorescence from only one metallic nano-well 3 was guided to a CCD detector cooled with liquid nitrogen.

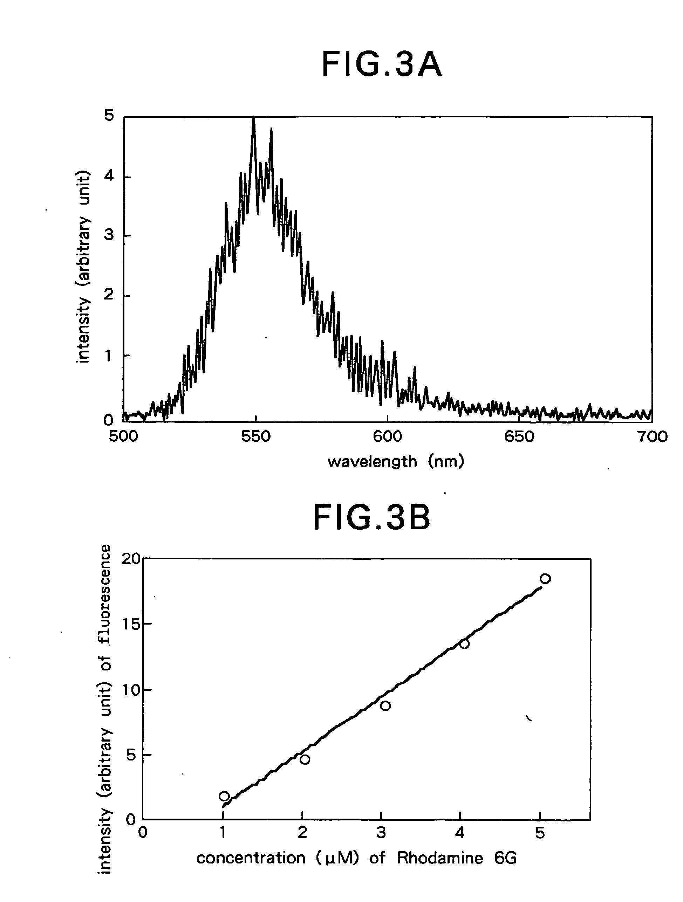

Fig. 3A shows spectrum as observation results, and Fig. 3B shows relationship between concentration of Rhodamine 6G as an antigen and intensity of fluorescence. It is noted from Figs. 3A and 3B that emission of fluorescence from a nanometer zone is clearly observed with linear relationship between concentration of the antigen and intensity of fluorescence. This result provesthat immunoassay in the metallic nano-well 3 is applicable for practical use.

### Example 2

A modified metallic nano-well is applied to genetic analysis in this example.

### Preparation of metallic nano-wells

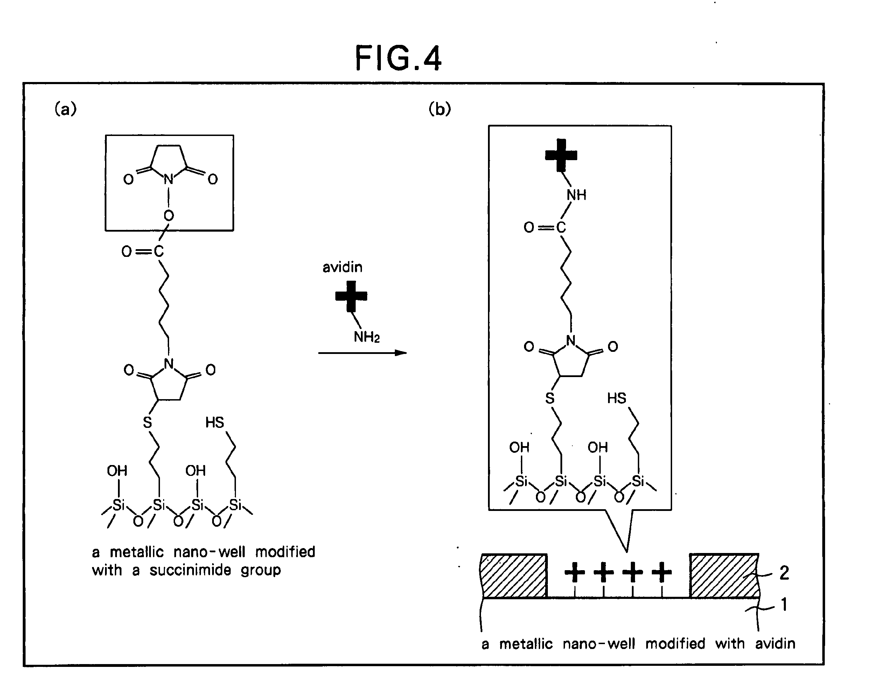

A metallic nano-well 3 having a bottom modified with a thiol group in the same way as Example 1 was held in contact with succinimidyl-6-maleimidylhexanoate, so as to further modify the bottom of the metal nano-well 3 with a succinimidyl group (indicated by (a) in Fig. 4).

### Modification of bottom of metallic nano-well

The succinimidyl group on the bottom of the metal nano-well 3 was reacted with an amino group on avidin by immersion of the processed metal film 2 in an avidin liquid, so as to modify the bottom of the metal nano-well 3 with avidin (indicated by (b) in Fig. 4).

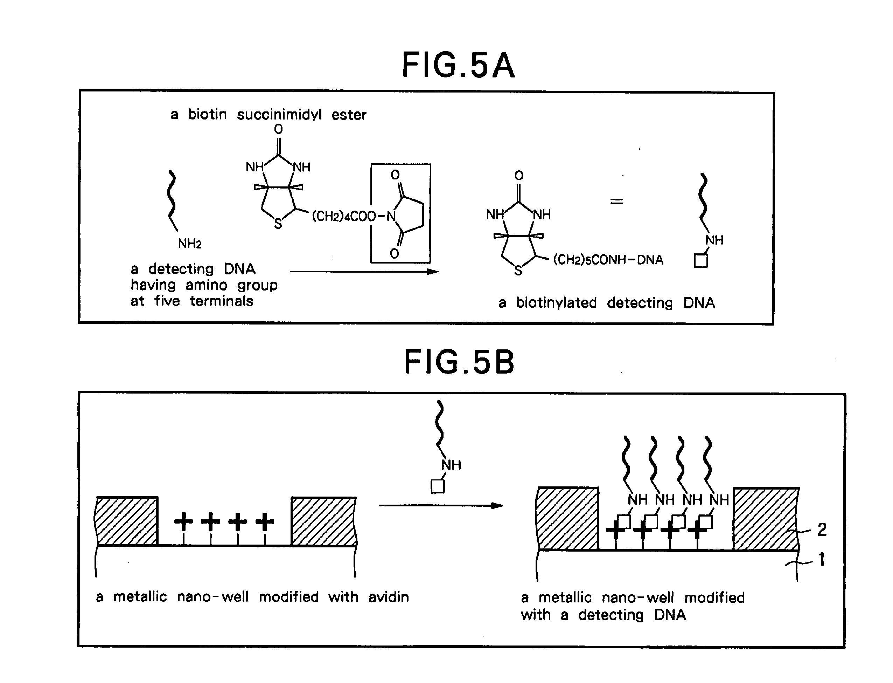

TS oligonucleotide was used as DNA for detection of hybridization. A DNA was prepared by providing 13-mer oligonucleotide having 12 bases in complementary relation with TS oligonucleotide, adding amino groups to five terminals of the 13-mer oligonucleotide, and reacting the 13-mer oligonucleotide with a biotin succinimidyl ester as a biotinylation reagent to modify the five terminals with biotin (as shown in Fig. 5A).

Of course, the DNA is not limited to TS oligonucleotide, but any other DNAs may be used for detection of hybridization. Kinds of the biotinylation reagent and the DNA are properly determined in relation with the DNA, instead of the biotin succinimidyl ester and the DNA having 12 bases.

Due to reactivity of avidin with biotin, a bottom of the metallic nano-well 3 was well modified with the DNA by immersing the metal film 2 having the metallic nano-well 3 modified with avidin in a 5ng/ml solution of the biotinylated DNA for 3 minutes (as shown in Fig. 5B). Modification of the DNA was dependent on reaction of avidin with biotin in this example, but the DNA can be directly coupled with a thiol group.

### Hybridization

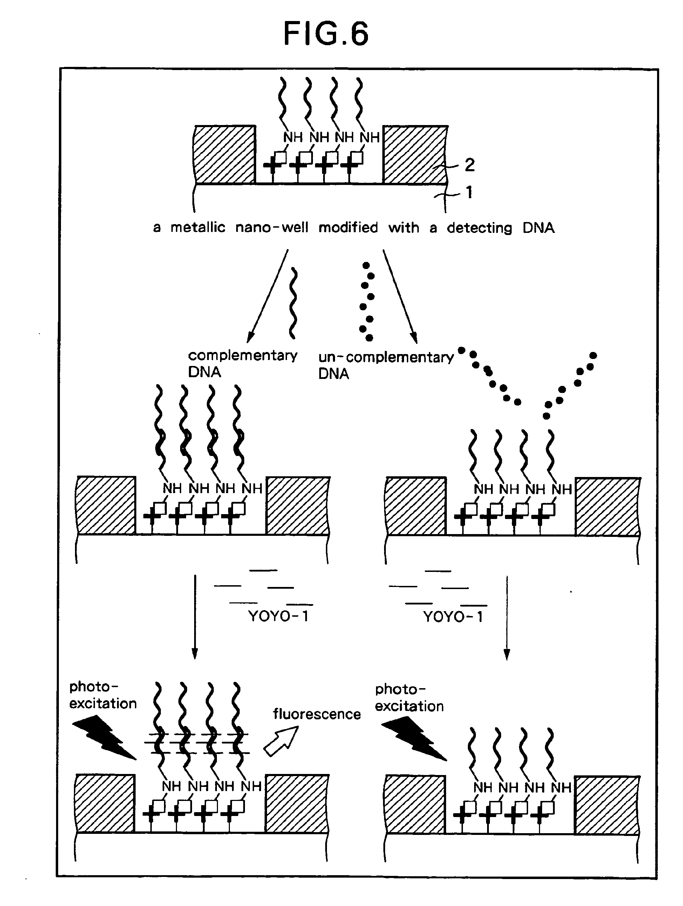

Two quartz substrates 1, each of which had a bottom modified with the detecting DNA, were prepared. One quartz substrate 1 was immersed 30 minutes in a 30 ng/ml solution of TS oligonucleotide, and the other quartz substrate 1 was immersed 30 minutes in a 30ng/ml solution of control oligonucleotide which was absolutely un-complementary with the detecting DNA. The objective DNA, which was complementary with the detecting DNA, was introduced in the metallic nano-well 3 by hybridization, but the other DNA, which was not complementary with the detecting DNA, was not hybridized (as shown in Fig. 6).

Thereafter, each quartz substrate 1 was immersed 10 minutes in a 5ng/ml solution of YOYO-1, i.e. a fluorescent reagent which peculiarly coupled with double-chained DNA YOYO-1 coupled with the objective DNA hybridized in the metallic nano-well 3, but YOYO-1 did not bond to the unhybridized metallic nano-well 3.

Other fluorescent reagents for labeling DNA may be used instead of YOYO-1, as far as they peculiarly couple with the double-chained DNA Of course, the objective DNA may be direct labeled with the fluorescent reagent.

### Generation of surface plasmon electromagnetic field and observation of fluorescence

The quarts substrate 1, which had the metallic nano-wells 3 processed as above-mentioned, was fixed on a stage of a fluorescence microscope, and irradiated with Ar laser beam of 488 nm to generate a surface plasmon electromagnetic field. Fluorescence was emitted from YOYO-1 in the metallic nano-well 3 and observed by the fluorescence microscope. Observation results are shown as spectrum in Fi. 7. It is noted that emission of intense fluorescence was detected from the metallic nano-well 3 immersed in TS oligonucleotide in complementary relation with the DNA. But, fluorescence emitted from the metallic nano-well 3, which was immersed in control oligonucleotide without complementary with the DNA, was very weak. This comparison proves that genetic analysis in the metallic nano-well is realized due to vivid recognition of hybridization.

### INDUSTRIAL APPLICABILITY OF THE INVENTION

The inventive element for fluorescence analysis has an antibody or detecting DNA, which is reactive with antigen or objective DNA according to antigen-antibody reaction or hybridization, fixed to a bottom of a metallic nano-well present in a metal film. Fluorescence, which is emitted from the metal nano-well by application of a surface plasmon electromagnetic field, is detected or measured for immunity or genetic analysis. Since fluorescence is produced by application to the surface plasmon electromagnetic field to the metallic nano-well, a detecting reagent or sample is fixed without discoloration of fluorescence and excited with high efficiency, and measurement results are gained with extremely high sensitivity, compared with conventional fluorescence analysis. Consequently, the inventive element is useful in various fields such as biochemistry, clinical analysis and environmental analysis

## Claims

1. An element for fluorescence analysis, which comprises a metal film deposited on a surface of a substrate, nano-wells formed in said metal film and an active group or DNA fixed to a bottom of said nano-well, wherein fluorescence is emitted by photo-excitation of a fluorescence reagent bonded to an antigen coupled with said active group, or to an objective DNA hybridized with said nanowell fixed DNA, wherein the nano-wells have a diameter near wave-length of exciting light.

2. A method of manufacturing an element for fluorescence analysis, which comprises the steps of:
depositing a metal film on a surface of a substrate processed with a silane-coupling reagent,
forming minute holes, which serve as nano-wells, in said metal film, wherein the nano-wells have a diameter near wave-length of exciting light, and
modifying a bottom of said nano-wells with an active group or DNA.

## Patentansprüche

1. Fluoreszenzanalyseelement, welches einen auf einer Oberfläche eines Substrats abgeschiedenen Metallfilm, in dem Metallfilm gebildete Nanovertiefungen und eine an einem Boden der Nanovertiefung befestigte aktive Gruppe oder DNA umfasst,
wobei Fluoreszenz durch Photoanregung eines Fluoreszenz-Reagenzes emittiert wird, welches an ein Antigen gebunden ist, das mit der aktiven Gruppe oder einer mit der in der Nanovertiefung befestigten DNA hybridisierten Ziel-DNA gekoppelt ist,
wobei die Nanovertiefungen einen Durchmesser nahe der Wellenlänge des anregenden Lichtes aufweisen.

2. Verfahren zur Herstellung eines Fluoreszenzanalyseelementes, umfassend die Schritte:
des Abscheidens eines Metallfilms auf einer Oberfläche eines mit einem Silan-Kupplungsmittel behandelten Substrates,
des Formens winziger Löcher in dem Metallfilm, welche als Nanovertiefungen dienen, wobei die Nanovertiefungen einen Durchmesser nahe der Wellenlänge des anregenden Lichtes aufweisen, und
des Modifizierens eines Bodens der Nanovertiefungen mit einer aktiven Gruppe oder DNA.

## Revendications

1. Elément d'analyse par fluorescence, comprenant un film métallique déposé à la surface d'un substrat, des nanopuits formés dans ledit film métallique et un groupe actif ou de l'ADN fixé au fond dudit nanopuits, dans lequel la fluorescence est émise par photoexcitation d'un réactif fluorescent lié à un antigène couplé audit groupe actif, ou à un ADN d'intérêt hybridé avec ledit ADN fixé au fond du nanopuits, les nanopuits présentant un diamètre proche de la longueur d'onde de la lumière excitatrice.

2. Procédé de fabrication d'un élément d'analyse par fluorescence comprenant les étapes consistant à :
déposer un film métallique sur une surface d'un substrat traitée au moyen d'un réactif de couplage silane,
former de minuscules trous, servant de nanopuits, dans ledit film métallique, les nanopuits présentant un diamètre proche de la longueur d'onde de la lumière excitatrice,
modifier le fond desdits nanopuits au moyen d'un groupe actif ou d'un ADN.
